Europäisches Patentamt

European Patent Office

Office européen des brevets

(19)

(11) **EP 0 808 836 B1**

(12) **EUROPEAN PATENT SPECIFICATION**

(45) Date of publication and mention
of the grant of the patent:
**19.03.2003 Bulletin 2003/12**

(51) Int Cl.[7]: **C07D 251/60**, C07D 251/62

(21) Application number: **97201380.9**

(22) Date of filing: **07.05.1997**

(54) **Method for the preparation of melamine**

Verfahren zur Herstellung von Melamin

Procédé pour la préparation de mélamine

(84) Designated Contracting States:
**AT BE CH DE DK ES FI FR GB GR IT LI NL PT SE**

(30) Priority: **14.05.1996 NL 1003105**

(43) Date of publication of application:
**26.11.1997 Bulletin 1997/48**

(73) Proprietor: **DSM N.V.**
**6411 TE Heerlen (NL)**

(72) Inventors:
• **van Wijck, Julius, Gerardus, Theodorus**
**6211 SL Maastricht (NL)**

• **de Haan, André, Banier**
**6137 JW Sittard (NL)**
• **Biermans, Andreas, Johannes**
**6129 GS Stein (NL)**
• **Hardeveld van, Rudolf**
**6191 BA Beek (NL)**

(56) References cited:
**EP-A- 0 747 366**         **WO-A-96/20182**
**WO-A-96/23778**          **US-A- 4 565 867**

**Description**

BACKGROUND OF THE INVENTION

1. Field of the Invention

The invention relates to a method for the recovery and purification of melamine from urea in which melamine is recovered from a urea pyrolysis reaction product comprised of melamine by mixing the reaction product with a medium, pressurizing the resulting mixture, and then expanding the pressurized mixture whereupon the medium evaporates and a dry solid very high purity melamine is produced. More particularly, the invention relates to a method for the recovery and purification of melamine from urea via a non-catalytic, high-pressure process in which melamine is recovered from a melamine melt leaving the melamine reactor vessel by mixing the melamine melt with a liquid medium, in particular ammonia, pressurizing the resulting mixture, and then expanding the pressurized mixture which evaporates the liquid medium and produces dry solid high purity melamine. The product thus obtained is commercially usable.

2. Description of the Related Art

A continuous, anhydrous, non-catalytic, high-pressure process for the production of melamine from urea is described in U.S. Patent No. 4,565,867. The patent describes the pyrolysis of urea in a reactor at a pressure of about 10.3 MPa to 17.8 MPa and a temperature of about 354°C to 427°C to produce a reaction product which contains liquid melamine, $CO_2$ and $NH_3$ and is transferred under pressure, as a mixed stream, to a gas-liquid separator unit. In the gas-liquid separator unit, which is kept at virtually the same pressure and temperature as the reactor, the reaction product is separated into gaseous and liquid streams. The gaseous stream contains $CO_2$ and $NH_3$ off-gases and also melamine vapor while the liquid stream consists substantially of liquid melamine. The gaseous product is sent to a gas scrubber, while the liquid melamine is transferred to a product cooler. In the gas scrubber the above-mentioned $CO_2$ and $NH_3$ off-gases and melamine vapor, are scrubbed, at virtually the same pressure as the reactor pressure, with molten urea so as to pre-heat the urea, cool said off-gases and remove the melamine. The pre-heated urea, which contains the removed melamine, is then fed to the reactor. Meanwhile, in the product cooler, the pressure and temperature of the liquid melamine from the gas-liquid separator are reduced by means of a liquid cooling medium, preferably liquid ammonia, so as to produce a solid melamine product.

The drawback of this method is that the purity of the melamine obtained is typically in the range of 96 to 99.5%. Besides melamine, contaminants such as urea, $CO_2$, ammeline-related compounds and other organic solids (melem and melam, for example) are present. The resulting purity of such a product is not high enough for some critical melamine applications, such as coatings. There is a need for a method to directly obtain, without further purification steps, higher purity melamine.

SUMMARY OF THE INVENTION

An object of the present invention is an improved method for the preparation of very high purity melamine, generally from 99.5 to 99.95 wt.%, from urea in which the melamine is recovered from the urea pyrolysis reaction product as a dry powder. One particular object is an improved, continuous, high-pressure, non-catalytic process for the production of melamine from urea in which very high purity melamine is obtained directly as a dry powder from the melamine melt through cooling. These and other objects are achieved by the cooling of a urea reaction product comprising melamine by separating the urea reaction product into gaseous and liquid phases with the liquid phase comprising melamine. The liquid phase is then transferred to a mixing vessel where it undergoes mixing with a liquid medium to produce a liquid mixture at temperatures and pressures such that the formation of solid melamine is substantially avoided. Optionally, the liquid mixture is pressurized. Then the liquid mixture, further pressurized or not, is expanded thereby cooling the liquid mixture, evaporating the liquid medium, and obtaining a solid very high purity melamine product. Liquid ammonia is the preferred liquid medium.

Another method for the preparation of very high purity melamine from urea is via a melamine melt and is accomplished by separately pressurizing both a melamine melt and a liquid medium then mixing the pressurized melamine melt and pressurized liquid medium in a mixing vessel to produce a pressurized liquid mixture. This pressurized liquid mixture is then expanded into an expansion vessel whereby the liquid medium evaporates and the desired melamine product is obtained as a dry material.

WO 96 20182, published after the filing date of the present invention, discloses a process for producing high-purity melamine. The process of WO 96 20182 comprises the steps of removing the $NH_3/CO_2$ gas mixture from the liquid melamine, reducing the dissolved $CO_2$ by introducing gaseous ammonia, resting the liquid phase, followed by controlled slow cooling under pressure to a temperature between 270°C and 330°C.

WO 96 23778, published after the filing date of the present invention, discloses a process for the purification of melamine. The process of WO 96 23778 comprises the step of keeping unpurified melamine for from 5 minutes to 20 hours at a partial ammonia pressure of 15 to 40 MPa within a temperature range from 280°C to 430°C, followed by cooling and depressurising.

EP-A-0 747 366 and US-A-5 514 796 disclose a process for production of melamine of improved purity by a high-pressure, non-catalytic process. The process of EP-A-0 747 366 and US-A-5 514 796 comprises the step of depressurising and quenching in a cooling unit a liquid melamine stream with a liquid medium which will form a gas at the temperature of the liquid melamine. Said cooling unit has a pressure in excess of 4.14 MPa (600 psi).

BRIEF DESCRIPTION OF THE DRAWINGS

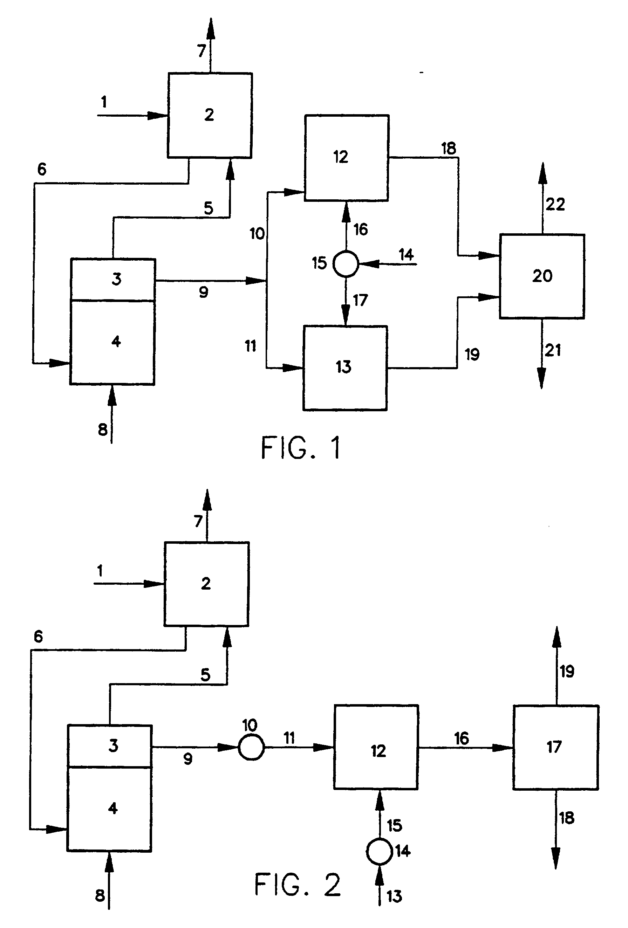

Figures 1 through 4 are flow diagrams of different embodiments of a system for the production of melamine from urea according to the present invention.

DETAILED DESCRIPTION OF THE INVENTION

We have now found that the purity of the melamine can be substantially increased by combining the urea pyrolysis liquid reaction product comprising melamine with a liquid medium, optionally increasing the pressure of the resulting mixture, and subsequently expanding the resulting liquid mixture, thereby evaporating the liquid medium and obtaining the melamine as a dry material. The liquid medium can be at the same, or a higher or lower temperature than the liquid melamine, preferably lower. The resulting liquid mixture can be cooled to just above its solidification point prior to expansion. Preferably ammonia is used as the liquid medium.

More particularly we have discovered that substantial increases in the purity of the melamine can be obtained in the preparation of melamine from urea via a non-catalytic, high-pressure process, in which melamine is recovered from the liquid melamine melt leaving the reactor vessel, by combining the melamine melt with a liquid medium, optionally increasing the pressure of the resulting liquid mixture, and subsequently expanding the liquid mixture, thereby evaporating the liquid medium and obtaining melamine as a dry material. Prior to expansion, the temperature and pressure conditions of the liquid mixture are sufficient to prevent the formation of solid melamine. The liquid melamine melt leaving the reactor vessel can be combined with a liquid medium at the same or a lower or higher temperature. Preferably, the temperature of the liquid medium is lower than the liquid melamine melt. The resulting liquid mixture can be cooled to just above its solidification point prior to expansion. Preferably ammonia is used as the liquid medium. This method yields melamine having a purity, without further washing or purification, of greater than about 99 wt.%, and more specifically having a purity of about 99.5 to about 99.95 wt. %.

Urea, preferably in the form of a melt, is the preferred starting material for the production of melamine. Ammonia and carbon dioxide are by-products obtained during melamine preparation, which proceeds according to the following reaction equation:

$$6 \ CO(NH_2)_2 \rightarrow C_3N_6H_6 + 6 \ NH_3 + 3 \ CO_2$$

The preparation of the melamine can be carried out at high pressure, preferably between about 5 and about 100 MPa, without a catalyst. The temperature of the reaction varies between about 325°C and about 450°C and is preferably between about 350°C and about 425°C. The ammonia and carbon dioxide by-products are generally returned to an adjoining urea plant.

The results reported herein can be obtained in a plant suited for the preparation of melamine from urea. A plant suitable for the present invention can comprise a gas scrubber, a reactor vessel, optionally in combination an internal gas-liquid separator or with a separate gas-liquid separator, one or more mixing vessels, an expansion vessel, and optionally a $CO_2$ removal unit, a post-reactor vessel or an aging vessel.

In an embodiment of the method, melamine can be prepared from urea in a plant comprising a gas scrubber, a reactor vessel which contains an internal gas/liquid separator, two mixing vessels and an expansion vessel. Urea melt is fed to the gas scrubber at a pressure of about 5 to about 100 MPa, preferably about 8 to about 20 MPa, and at a temperature above the melting point of urea. This gas scrubber can be provided with a cooling jacket so as to ensure extra cooling. The gas scrubber can also be provided with internal cooling bodies. In the gas scrubber the liquid urea comes into contact with off-gases from the melamine reactor vessel or from a separate gas-liquid separator installed downstream of the reactor. The off-gases are composed primarily of $CO_2$ and ammonia with small amounts of melamine vapor. The urea melt scrubs the melamine vapor out of the off-gas and carries this melamine back to the reactor vessel.

In the scrubbing process the off-gases are cooled down from about the temperature of the reactor, that is from about 350°C to about 425°C, to about 175°C to 235°C, while the urea melt is heated to about 175°C to about 235°C. Below the above-mentioned minimum temperatures ammonia and carbon dioxide can condense in the bottom of the gas scrubber, which can result in the formation of ammonium carbamate, which can adversely affect the process. In order to prevent the detrimental formation of urea decomposition and/or condensation products, the gas scrubber temperature should, in general, not exceed about 275°C. The carbon dioxide and ammonia waste gases are removed from the top of the gas scrubber and are preferably returned to a urea plant for use as starting material.

The urea and melamine melt can then be withdrawn from the gas scrubber and fed, for example via a high-pressure pump, to the reactor vessel, which is at a pressure of about 5 to about 100 MPa, and preferably of about 8 to about 20 MPa. Gravity transfer of the urea melt can also be utilized by placing the gas scrubber above the reactor vessel.

In the reactor vessel the molten urea is converted into melamine, carbon dioxide and ammonia by heating to a temperature of about 325°C to about 450°C, preferably of about 350°C to about 425°C, at a pressure of about 5 to about 25 MPa, preferably of about 8 to about 20 MPa.

Ammonia can be fed to the reactor vessel in the form of a liquid or a hot vapor. The ammonia feed can serve as a purification agent to prevent clogging of the reactor vessel bottom or to prevent the formation of melamine condensation products such as melam, melem and melon, or to promote mixing in the reactor vessel. The amount of ammonia fed to the reactor is about 0 to about 10 mol per mol urea; preferably, about 0 to about 5 mol per mol urea, and in particular about 0 to about 2 mol per mol urea. The carbon dioxide and ammonia formed in the reaction as well as any extra ammonia feed into the reactor vessel can collect in an internal gas-liquid separation unit, for example in the top of the reactor vessel, or optionally in a separate gas-liquid separator downstream of the reactor vessel, and can be separated, in gaseous form, from the liquid melamine. As described above, the resulting gas mixture of carbon dioxide, ammonia and melamine is sent to the gas scrubber for removal of the melamine vapor and to preheat the urea melt. The liquid melamine is withdrawn from the reactor vessel and transferred to one or more mixing vessels.

Ammonia is added to the melamine melt in the mixing vessels and an ammonia pump is utilized to increase the pressure to about 10 to about 100 MPa, preferably about 20 to about 90 MPa and more preferably above 30 MPa. The mixing vessels are preferably equipped with means for the preparation of a melamine/ammonia mixture. The temperature in the mixing vessels can be the same as in the reactor, but can also be reduced by the ammonia, and preferably is reduced from the reactor temperature to about 1°C to about 50°C, and more preferably about 1°C to about 20°C, and more particularly about 1°C to about 10°C above the solidification point of the mixture. This cooling has the effect of converting the condensation products of melamine back into melamine and also permitting quicker solidification during the subsequent expansion, since some of the heat has already been removed. Depending on the pressure, a mixture of melamine and ammonia will contain from about 10 to about 60 wt.% dissolved ammonia, generally the amount of dissolved ammonia is greater than 10 wt.%.

Subsequently, this pressurized solution is alternately sprayed from one or the other mixing vessel into an expansion vessel. A semi-continuous flow into the expansion vessel can thus be established since the contents of one of the mixing vessels can be transferred to the expansion vessel while the other vessel is filled with melamine and pressurized with ammonia. The residence time of the mixture in a mixing vessel is between about 10 seconds and about 5 hours. Preferably, residence time is between about 1 minute and about 60 minutes.

The pressure and temperature of the expansion vessel are about 0 MPa to about 10 MPa and about 0°C to about 250°C, respectively. Preferably, the temperature is about 10°C to about 100°C. During the expansion step the evaporation of ammonia causes the temperature to drop, so that the heat of crystallization of the melamine can be discharged, yielding melamine in the form of a solid powder. Since the melamine/ammonia mixture that is being expanded is by preference a homogeneous mixture, very fast homogeneous cooling takes place, so that few by-products are formed. The evaporated ammonia is recycled and reintroduced into the process.

The melamine powder thus produced has a melamine content generally greater than about 99 wt. %, and more specifically a melamine content of about 99.5 to about 99.95 wt.% and can be employed, generally, without further purification, in applications which require very high purity melamine.

In yet another preferred embodiment of the method, instead of two mixing vessels, use is made of only one mixing vessel with a melamine pump upstream of it. The melamine pump is used, prior to the mixing vessel, to increase the pressure of the melamine to between about 10 and about 100 MPa preferably about 20 to about 90 MPa and more preferably above 30 MPa. In the mixing vessel the high pressure melamine is combined with ammonia at virtual the same high pressure. This method results in a continuous flow of the homogeneous melamine/ammonia solution from the mixing vessel to the expansion vessel.

In another preferred embodiment of the method, the liquid melamine is treated in a $CO_2$ removal unit and/or a post-reactor or an aging vessel before being transferred to the expansion vessel. The aging vessel can be used in conjunction with or in place of the mixing vessel(s). In the aging vessel the liquid melamine can be contacted with about 0.01 to about 10 mol ammonia per mol melamine, and preferably about 0.1 to about 2 mol ammonia per mol melamine. The contact time in the aging vessel is between about 1 minute and about 3 hours, preferably between about 2 minutes and about 1 hour. The temperature and pressure in the aging vessel are virtually the same as in the reactor vessel. The ammonia leaving the aging vessel can subsequently be sent to the reactor vessel. In the aging vessel the melamine condensation products are converted back into melamine. The aging vessel may be positioned directly downstream of the melamine reactor vessel or between the mixing vessels and the expansion vessel. In the $CO_2$ removal unit the melamine is stripped with ammonia to remove any $CO_2$ residues. This step further increases the purity of the melamine, in particular by preventing the formation of ammeline, ammelide and cyanuric acid. The $CO_2$ removal unit is placed directly downstream of the melamine reactor vessel.

In another embodiment of the method, the melamine is synthesized at such a high pressure that after gas-liquid separation the melamine melt and the liquid ammonia can be sent directly to the expansion vessel. The pressure in the melamine reactor and the gas-liquid separator in this embodiment is between about 60 and about 100 MPa. In this embodiment, the ammonia is metered into the melamine reactor vessel to obtain the melamine/ammonia mixture which is subsequently expanded.

The flow diagram of Figure 1 illustrates an embodiment of the present invention. In this embodiment, liquid urea is fed to a gas scrubber 2 via line 1. Via line 5, a gas stream consisting of $NH_3$, $CO_2$ and melamine is fed from gas-liquid separator 3, which is combined with synthesis zone 4, to gas scrubber 2. In this gas scrubber 2 the melamine is scrubbed from the gas stream by means of the urea stream supplied via line 1. Via line 6 the urea with the melamine enters the synthesis zone 4. A gas stream containing $NH_3$ and $CO_2$ leaves the gas scrubber 2 via line 7 to an adjoining urea plant. Via line 8 $NH_3$ can be fed to synthesis zone 4. In synthesis zone 4 the urea is heated to produce melamine, in both liquid and gaseous states, gaseous carbon dioxide, and gaseous ammonia. The gaseous and liquid phases are separated in the separator 3. The liquid melamine stream from separator 3 is alternately passed to two mixing vessels, 12 and 13, via lines 9, 10 and 11. Via line 14 $NH_3$ is supplied to pump 15 and heated and pressurized to the desired levels and then fed via lines 16 and 17 to mixing vessels 12 and 13 to produce a melamine/ammonia solution. Then, the melamine/ammonia solution is sent alternately, via lines 18 and 19, from mixing vessels 12 and 13 to expansion vessel 20, where its pressure and temperature are reduced. From this expansion vessel 20 the resulting solid very high purity melamine powder is discharged via line 21, and via line 22 the gaseous $NH_3$ is discharged to lines 8 and 14.

Figure 2 illustrates a second embodiment of the invention. In this embodiment, liquid urea is fed to a gas scrubber 2 via line 1. Via line 5, a gas stream consisting of $NH_3$, $CO_2$ and melamine is fed from gas-liquid separator 3, which is combined with synthesis zone 4, to gas scrubber 2. In this gas scrubber 2 the melamine is scrubbed from the gas stream by means of the urea stream supplied via line 1. Via line 6 the urea with the melamine enters the synthesis zone 4. A gas stream containing $NH_3$ and $CO_2$ leaves the gas scrubber 2 via line 7 to an adjoining urea plant. Via line 8 $NH_3$ can be fed to synthesis zone 4. In synthesis zone 4 the urea is heated to produce melamine, in both liquid and gaseous states, gaseous carbon dioxide, and gaseous ammonia. The gaseous and liquid phases are separated in the separator 3. The liquid melamine stream from separator 3 is sent to a melamine pump 10 via line 9. After its pressure has been increased the liquid melamine is sent to a mixing vessel 12 via line 11. Simultaneously, via line 13 $NH_3$ is passed to pump 14. After its pressure has been increased, the $NH_3$ is passed to mixing vessel 12 via line 15 to produce a melamine/ammonia solution. Via line 16 the melamine/ammonia solution is fed to expansion vessel 17, where its pressure and temperature are reduced. Via line 18, the resulting solid very high purity melamine powder is discharged from the expansion vessel 17, and via line 19 the gaseous $NH_3$ is discharged to lines 8 and 13.

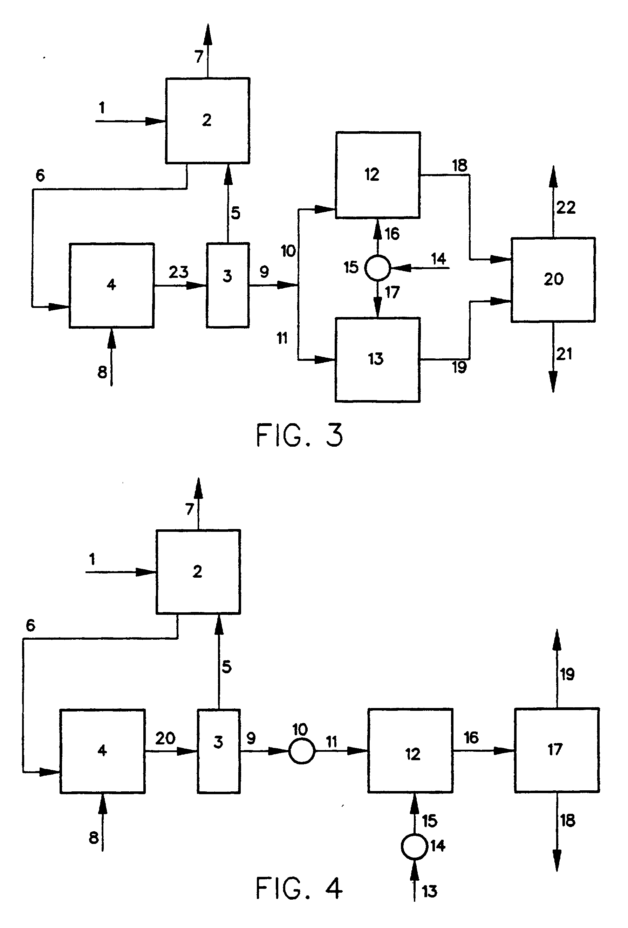

The embodiments as shown in Figures 1 and 2 and described above may also involve the use of a separate separation unit 3, as shown in Figures 3 and 4 instead of a combined reactor/separator unit. As shown in Figures 3 and 4, the gas-liquid stream from synthesis zone 4 is fed to separator 3 via lines 23 and 20 respectively. The melamine melt leaving separator 3 is treated in a similar fashion as shown in Figures 1 and 2.

Further embodiments of the present invention can also be realized using a post-reactor, an aging vessel or a $CO_2$ removal unit positioned directly downstream of the reactor/separator in line 9 or, if use is made of a post-reactor or an aging vessel, between the mixing vessel or, as the case may be, mixing vessels and the expansion vessel.

This method for the preparation of very high purity melamine has been described in patent application number 1003105 filed in The Netherlands on the date of May 14, 1996.

The following non-limiting example further describes the present invention.

EXAMPLE

From a melamine reactor 100 g of melamine melt was transferred to an autoclave, where it was combined with 100 g of ammonia. The pressure of the autoclave contents was raised to 80 MPa at a temperature of $250°C$ for 45 minutes. The mixture was then expanded to a pressure of 3 MPa. Melamine powder having a purity of 99.8 wt.% was obtained.

**Claims**

1. A process for the recovery of melamine from a urea reaction product which comprises melamine, said process comprising the steps of:

a) separating the urea reaction product into a first gaseous phase and a liquid phase, wherein the liquid phase comprises liquid melamine;
b) transferring the liquid phase to a first vessel at a temperature of between $325°C$ and $450°C$ and a pressure of between 5 MPa and 25 MPa;
c) mixing in the first vessel the liquid phase with an ammoniacal liquid medium and thereby producing a liquid mixture at a pressure of between 10 MPa and 100 MPa and a temperature of between 1°C and 50°C above the solidification point of the liquid mixture at the prevailing pressure; and

d) expanding the liquid mixture into a second vessel and thereby evaporating the liquid medium and obtaining the melamine as a dry solid material.

2. A process according to claim 1, wherein the liquid medium includes ammonia.

3. A process according to claim 1, further comprising reducing the temperature of the liquid mixture in the first vessel from an initial temperature to 1 C to 20 C above the solidification point of the liquid mixture.

4. A process according to claim 1, wherein said mixing step is performed in at least two vessels.

5. A process for the recovery of solid melamine from a melamine melt, said process comprising the steps of:

a) pressurizing a melamine melt;
b) pressurizing an ammoniacal liquid medium;
c) mixing in a first vessel the pressurized melamine melt and the pressurized liquid medium and thereby producing a pressurized liquid mixture at a pressure of between 10 MPa and 100 MPa and a temperature of between 1°C and 50°C above the solidification point of the liquid mixture at the prevailing pressure; and
d) expanding the pressurized liquid mixture into a second vessel and thereby depressurizing the liquid mixture, evaporating the liquid medium and obtaining the solid melamine as a dry material.

6. A process according to claim 5, wherein said liquid medium includes ammonia.

7. A process according to claim 5, wherein said step of pressurizing the melamine melt involves pressurizing the melamine melt to 10 MPa to 100 MPa.

8. A process according to claim 5, wherein said step of pressurizing the liquid medium involves pressurizing the liquid medium to 10 MPa to 100 MPa.

9. A process for the recovery of melamine from a urea reaction product which comprises melamine, said process comprising the steps of:

a) separating the urea reaction product into a gaseous phase and a liquid phase, wherein the liquid phase comprises liquid melamine;

b) transferring the liquid phase to one or more mixing vessels at a temperature of between 325°C and 450°C and a pressure of between 5 MPa and 25 MPa;
c) mixing in said mixing vessel or vessels said liquid phase with an ammoniacal liquid medium and thereby producing a liquid mixture at a pressure of between 10 MPa and 100 MPa and a temperature of between 1°C and 50°C above the solidification point of the liquid mixture at the prevailing pressure; and
d) expanding the liquid mixture from the mixing vessel or vessels into an expansion vessel and thereby evaporating the liquid medium and obtaining the melamine as a dry solid material.

**Patentansprüche**

1. Verfahren für die Gewinnung von Melamin aus einem Harnstoff-Umsetzungsprodukt, welches Melamin umfasst, wobei besagtes Verfahren die Schritte umfasst:

a) Trennen des Harnstoff-Umsetzungsprodukts in eine erste gasförmige Phase und eine flüssige Phase, wobei die flüssige Phase flüssiges Melamin umfasst;
b) Übertragen der flüssigen Phase auf ein erstes Gefäß bei einer Temperatur von zwischen 325°C und 450°C und einem Druck von zwischen 5 MPa und 25 MPa;
c) Mischen der flüssigen Phase mit einem ammoniakhaltigen flüssigem Medium im ersten Gefäß und dadurch Herstellen eines flüssigen Gemisches bei einem-Druck von zwischen 10 MPa und 100 MPa und einer Temperatur von zwischen 1°C und 50°C über dem Erstarrungspunkt des flüssigen Gemisches beim vorherrschenden Druck; und
d) Expandieren des flüssigen Gemisches in ein zweites Gefäß und dadurch Eindampfen des flüssigen Mediums und Erhalten des Melamins als ein trockenes, festes Material.

2. Verfahren gemäß Anspruch 1, wobei das flüssige Medium Ammoniak einschließt.

3. Verfahren gemäß Anspruch 1, welches ferner Verringern der Temperatur des flüssigen Gemisches im ersten Gefäß von einer Anfangstemperatur auf 1°C bis 20°C über dem Erstarrungspunkt des flüssigen Gemisches umfasst.

4. Verfahren gemäß Anspruch 1, wobei besagter Mischungsschritt in mindestens zwei Gefäßen durchgeführt wird.

**5.** Verfahren für die Gewinnung von festem Melamin aus einer Melaminschmelze, wobei besagtes Verfahren die Schritte umfasst:

a) Unter-Druck-Setzen einer Melaminschmelze;

b) Unter-Druck-Setzen eines ammoniakhaltigen flüssigen Mediums;

c) Mischen der unter Druck gesetzten Melaminschmelze und des unter Druck gesetzten flüssigen Mediums in einem ersten Gefäß und dadurch Herstellen eines unter Druck gesetzten, flüssigen Gemisches bei einem Druck von zwischen 10 MPa und 100 MPa und einer Temperatur von zwischen 1°C und 50°C über dem Erstarrungspunkt des flüssigen Gemisches beim vorherrschenden Druck; und

d) Expandieren des unter Druck gesetzten flüssigen Gemisches in ein zweites Gefäß und dadurch Ablassen des Drucks des flüssigen Gemisches, Abdampfen des flüssigen Mediums und Erhalten des festen Melamins als ein trockenes Material.

**6.** Verfahren gemäß Anspruch 5, wobei besagtes flüssiges Medium Ammoniak einschließt.

**7.** Verfahren gemäß Anspruch 5, wobei besagter Schritt des Unter-Druck-Setzens der Melaminschmelze Unter-Druck-Setzen der Melaminschmelze auf 10 MPa bis 100 MPa einbezieht.

**8.** Verfahren gemäß Anspruch 5, wobei besagter Schritt des Unter-Druck-Setzens des flüssigen Mediums Unter-Druck-Setzen des flüssigen Mediums auf 10 MPa bis 100 MPa einbezieht.

**9.** Verfahren für die Gewinnung von Melamin aus einem Harnstoff-Umsetzungsprodukt, welches Melamin umfasst, wobei besagtes Verfahren die Schritte umfasst:

a) Trennen des Harnstoff-Umsetzungsprodukts in eine gasförmige Phase und eine flüssige Phase, wobei die flüssige Phase flüssiges Melamin umfasst;

b) Übertragen der flüssigen Phase auf ein oder mehrere Mischgefäße bei einer Temperatur von zwischen 325°C und 450°C und einem Druck von zwischen 5 MPa und 25 MPa;

c) Mischen der besagten flüssigen Phase mit einem ammoniakhaltigen flüssigem Medium in besagtem Mischgefäß oder Gefäßen und dadurch Herstellen eines flüssigen Gemisches bei einem Druck von zwischen 10 MPa und 100 MPa und einer Temperatur von zwischen 1°C und 50°C über dem Erstarrungspunkt des flüssigen Gemisches beim vorherrschenden Druck; und

d) Expandieren des flüssigen Gemisches aus dem Mischgefäß oder Gefäßen in ein Expansionsgefäß und dadurch Abdampfen des flüssigen Mediums und Erhalten des Melamins als ein trockenes, festes Material.

**Revendications**

**1.** Procédé pour la récupération de mélamine à partir d'un produit de réaction d'urée comprenant de la mélamine, ledit procédé comprenant les étapes consistant à :

a) séparer le produit de réaction d'urée en une première phase gazeuse et en une phase liquide, dans lequel la phase liquide comprend de la mélamine liquide ;

b) transférer la phase liquide vers un premier récipient à une température comprise entre 325°C et 450°C et à une pression comprise entre 5 MPa et 25 MPa ;

c) mélanger dans le premier récipient la phase liquide avec un milieu liquide ammoniacal et produire de cette façon un mélange liquide à une pression comprise entre 10 MPa et 100 MPa et à une température comprise entre 1°C et 50°C au-dessus du point de solidification du mélange liquide à la pression dominante ; et

d) dilater le mélange liquide dans un second récipient et évaporer de cette façon le milieu liquide et obtenir la mélamine sous forme de matière sèche solide.

**2.** Procédé selon la revendication 1, dans lequel le milieu liquide comprend de l'ammoniaque.

**3.** Procédé selon la revendication 1, comprenant en outre la réduction de la température du mélange liquide dans le premier récipient à partir d'une température initiale de 1°C à 20°C au-dessus du point de solidification du mélange liquide.

**4.** Procédé selon la revendication 1, dans lequel ladite étape de mélange est réalisée dans au moins deux récipients.

**5.** Procédé pour la récupération de mélamine solide à partir d'une matière fondue à base de mélamine, ledit procédé comprenant les étapes consistant à :

a) mettre sous pression une masse fondue à base de mélamine ;

b) mettre sous pression un milieu liquide ammoniacal ;

c) mélanger dans un premier récipient la masse fondue à base de mélamine sous pression et le milieu liquide sous pression et à produire de cette façon un mélange liquide sous pression à une pression comprise entre 10 MPa et 100 MPa et à une température comprise entre 1°C et 50°C au-dessus du point de solidification du mélange liquide à la température dominante ; et

d) dilater le mélange liquide sous pression dans un second récipient et à abaisser de cette façon la pression du mélange liquide, à évaporer le milieu liquide et à obtenir la mélamine solide sous forme de matière sèche.

6. Procédé selon la revendication 5, dans lequel ledit milieu liquide comprend de l'ammoniaque.

7. Procédé selon la revendication 5, dans lequel ladite étape de mise sous pression de la masse fondue à base de mélamine nécessite une mise sous pression de la masse fondue à base de mélamine comprise entre 10 MPa et 100 MPa.

8. Procédé selon la revendication 5, dans lequel ladite étape de mise sous pression du milieu liquide nécessite une mise sous pression du milieu liquide comprise entre 10 MPa et 100 MPa.

9. Procédé de récupération de la mélamine à partir d'un produit de réaction à l'urée comprenant de la mélamine, ledit procédé comprenant les étapes consistant à :

   a) séparer le produit de réaction d'urée en une phase gazeuse et une phase liquide, dans lequel la phase liquide comprend de la mélamine liquide ;

   b) transférer la phase liquide vers un ou plusieurs récipients de mélange à une température comprise entre 325°C et 450°C et à une pression comprise entre 5 MPa et 25 MPa ;

   c) mélanger dans ledit récipient de mélange ladite phase liquide avec un milieu liquide ammoniacal et à produire de cette façon un mélange liquide à une pression comprise entre 10 MPa et 100 MPa et à une température comprise entre 1°C et 50°C au-dessus du point de solidification du mélange liquide à la pression dominante ; et

   d) dilater le mélange liquide du ou des récipients de mélange dans un récipient de dilatation et à évaporer de cette façon le milieu liquide et à obtenir la mélamine sous forme de matière sèche solide.

FIG. 1

FIG. 2

FIG. 3

FIG. 4